# EUROPÄISCHE PATENTSCHRIFT

(11) **EP 1 714 988 B1**
(45) Veröffentlichungstag und Bekanntmachung des Hinweises auf die Patenterteilung: **09.01.2008**
(21) Anmeldenummer: 06112175.2
(22) Anmeldetag: 03.04.2006
(51) Int. Cl.: C08F 226/06, C08F 6/12, A61K 9/20

(54) **Bindemittel für Tabletten mit hoher Festigkeit auf basis von feinteiligen Vinyllactampolymeren, deren Herstellung und Verwendung**
High-strength tablet binders based on finely divided vinyllactam polymers, their preparation and use
Liant à haute resistance à partir de fines particules de polymères vinyllactam pour comprimés, sa production et son utilisation

(30) Priorität: 20.04.2005 DE 102005018465
(43) Veröffentlichungstag der Anmeldung: 25.10.2006
(73) Patentinhaber: BASF Aktiengesellschaft, 67056 Ludwigshafen (DE)
(72) Erfinder: Kolter, Karl, 67117, Limburgerhof (DE); Ascherl, Hermann, 67246, Dirmstein (DE); Fussnegger, Bernhard, 67489, Kirrweiler (DE)

(56) Entgegenhaltungen:
- EP-A- 0 687 694
- EP-A- 0 715 848
- EP-A- 1 437 375

## Beschreibung

Die vorliegende Erfindung betrifft Bindemittel auf Basis von feinteiligen, pulverförmigen Vinyllactampolymere , wobei die Bindemittel eine mittlere Teilchengröße von bis zu 35 µm und eine Schüttdichte von bis zu 0.2 g/ml aufweisen. Bevorzugt sind Bindemittel in Form von hohlkugelförmigen Körpern oder Teilen solcher hohlkugelförmigen Körper mit einer Wanddicke < 3 µm, wobei das Verhältnis des Durchmessers zur Wanddicke > 10 beträgt. Weiterhin betrifft die Erfindung ein Verfahren zur Herstellung solcher Bindemittel-Partikel sowie deren Verwendung zur Herstellung von Tabletten mit hoher Festigkeit.

Bei der Herstellung von komprimierten Darreichungsformen werden in aller Regel Bindemittel eingesetzt, um die mechanischen Eigenschaften insbesondere die Bruchfestigkeit und die Friabilität zu verbessern. In der Regel existieren zwei Herstellungprozesse von Tabletten: die Feuchtgranulation und die Direkttablettierung. Nach diesen Anwendungen lassen sich auch die Bindemittel einteilen in Feuchtbindemittel und Trockenbindemittel. Bei den Trockenbindemitteln erfolgt die Anwendung wie der Name sagt in trockener Form, das heißt, es erfolgt keine Auflösung in einem Lösungsmittel. Die Direkttablettierung ist natürlich der kostengünstigere Prozess, da die einzelnen Komponenten lediglich gemischt werden müssen, aber häufig scheitern die Entwicklungen daran, dass kein effektives Trockenbindemittel zur Verfügung steht. Arzneistoffe und auch viele andere Tabletteneinsatzstoffe weisen häufig schlechte Tablettiereigenschaften auf, was insbesondere darauf zurückgeführt werden kann, dass keine Bindungen zwischen den Feststoffteilchen dieser Materialen bei der Komprimierung erzeugt werden können oder die Materialien so elastisch sind, dass bei der Rückdehnung die Bindungen wieder aufreißen. Prinzipiell könnte dies natürlich mit einem hohen Anteil an Bindemittel in der Tablette kompensiert werden. Allerdings ist dies nicht sinnvoll, weil dadurch die Masse und das Volumen der Tablette zunehmen und sie kaum mehr schluckbar ist. Außerdem verlängern hohe Bindemittelanteile die Zerfallszeit und Auflösung des Wirkstoffes. Viele Arzneistoffe können daher mittels Direkttablettierung nicht formuliert werden.

Die Trockenbindemittelwirkung spielt auch in der Walzenkompaktierung eine große Rolle, da auch hierbei ein starker Zusammenhalt zwischen den Teilchen der Tablettenbestandteile erzeugt werden muss. Ist dies nicht der Fall, entsteht eine mechanisch instabiles Walzenkompaktat, die bei der Zerkleinerung wieder nahezu in die Ausgangskorngröße zerfällt, schlecht fließt und bei der darauffolgenden Tablettierung keine ausreichende Bruchfestigkeit und Friabilität liefert.

Derzeit gibt es kein Trockenbindemittel mit ausreichenden Bindemitteleigenschaften, um diese Probleme zu lösen.

Häufig verwendete herkömmliche Bindemittel sind beispielsweise Vinyllactampolymere. Vinyllactampolymere sind kommerziell erhältlich und werden beispielsweise unter den Namen Kollidon® (BASF Aktiengesellschaft) und Plasdone® (International Speciality Products Inc.) vertrieben. Die mittlere Teilchengröße dieser Produkte liegt im Bereich von 50 bis 250 µm. Die Teilchen sind dickwandiger beziehungsweise von unregelmäßiger Struktur und weisen höhere Schüttdichten auf (Vgl. V. Bühler in "Polyvinypyrrolidone Excipients for Pharmaceuticals", S.18-20 und 186-188, Springer-Verlag Berlin Heidelberg, 2005).

Aus der EP-A 545209 ist die Herstellung von pulverförmigen wasserunlöslichen Vinylpyrrolidon-Vinylacetat-Copolymeren bekannt, die durch Versprühen wässriger Dispersionen erhalten werden.

Aus der EP-A 714 919 ist die Herstellung von pulverförmigen Polyvinylpyrrolidon-Wassserstoffperoxid-Komplexen durch Sprühtrocknung bekannt, wobei die Sprühtrocknung bei relativ niedrigen Drücken erfolgt.

Aus der EP-A 1 437 375 sind pulverförmige Vinylpyrrolidon-Polymere mit hoher Schüttdichte bekannt, die durch Versprühen mit Hilfe einer rotierenden Scheibe erhalten werden.

Aufgabe der vorliegenden Erfindung war es, Bindemittel auf Basis von Polyvinyllactamen mit verbesserten Bindemitteleigenschaften zur Herstellung von sehr festen Tabletten zu finden.

Demgemäß wurden Bindemittel auf Basis von feinteiligen pulverförmige VinyllactamPolymeren gefunden, die mittlere Teilchengrößen von 1 bis 35µm aufweisen.

Bevorzugt liegen diese Bindemittel als hohlkugelförmige Körper oder Teile solcher hohlkugelförmigen Körper vor, wobei die Wanddicke der hohlkugelförmigen Körper kleiner 3 µm und das Verhältnis des Durchmessers der hohlkugelförmigen Körper zur Wanddicke größer 10:1 1 beträgt.

Für die Bestimmung der mittleren Teilchengröße wird der D(4,3)-Wert unter Verwendung der Methode der Lichtbeugung herangezogen. Diese Teilchengröße soll kleiner 35 µm vorzugsweise kleiner 30 µm und besonders bevorzugt kleiner 20 µm liegen, bei einer Untergrenze der mittleren Teilchengröße von 2 µm .

Die Wanddicke der hohlkugelförmigen Körper beziehungsweise der Teile oder Bruchstücke solcher Körper soll vorzugsweise kleiner 3.0 µm betragen, insbesondere kleiner 2.5 µm und besonders bevorzugt kleiner 2.0 µm, bei einer Untergrenze von 0.05 µm, und das Verhältnis des Hohlkugeldurchmessers zur Schalendicke soll größer als 10 :1 bevorzugt größer 12:1 und besonders bevorzugt größer als 15:1 sein.

Die Schüttdichte beträgt weniger als 0,2 g/ml, insbesondere 0.05 bis 0.18 g/ml. Die Schüttdichte wird bestimmt nach Pharm. Eur. 2.9.15.

Die BET-Oberfläche liegt in der Regel oberhalb von 1 m² pro Gramm und kann bis zu 50 m²/g betragen.

Als Polyvinyllactame werden erfindungsgemäß wasserlösliche Homopolymere, Copolymere, Terpolymere, Blockcopolymere oder Pfropfcopolymere bezeichnet. Diese Polymere können als folgenden Monomere mit Lactamstruktur N-Vinylpyrrolidon oder N-Vinylcaprolactam oder Gemischen davon enthalten.

Geeignete weitere Comonomere sind die Vinylester der gesättigten C₁ - C₂₀₋Carbonsäuren wie z.B.: Vinylacetat, Vinylpropionat, Vinyllaurat, Vinylstearat.

Besonders bevorzugt sind wasserlösliche Copolymere aus Vinylpyrrolidon und Vinylacetat und hierbei ganz besonders bevorzugt Verhältnisse von Vinylpyrrolidon zu Vinylacetat von 60 : 40 bis 80 : 20, insbesondere Copolymere aus N-Vinylpyrrolidon und Vinylacetat im Gewichtverhältnis 6:4, K-Wert 25-30.

Bevorzugt sind weiterhin Homopolymere des N-Vinylpyrrolidons.

Die K-Werte nach Fikentscher, ein Mass für das Molekulargewicht der Polymere, können 10 bis 120, bevorzugt 12 bis 90, besonders bevorzugt 15 bis 60 betragen.

Bevorzugt sind erfindungsgemäß Bindemittel, die zu 100 Gew.-% aus Polyvinyllactam-Polymeren bestehen. Jedoch können gewünschtenfalls weitere Stoffe zugesetzt werden.

So können zur Verbesserung der Plastizität der Bindemittel weichmachende Stoffe zugesetzt werden.. Als weichmachende Stoffe können typische Weichmacher wie Triethylcitrat, Triacetin, Propylenglykol, Glycerin, Polyethylenglykol und ähnliche bekannte Stoffe eingesetzt werden,. Der Anteil dieser Stoffe im erfindungsgemäßen Bindemittel beträgt 0 bis 20 Gew.-%, bevorzugt kleiner 10 Gew.-%.

Weiterhin können die erfindungsgemäßen Bindemittel neben dem Vinyllactam-Polymer auch grenzflächenaktive Stoffe enthalten. Grenzflächenaktive Stoffe sind Stoffe, die an einer Grenzfläche die Grenzflächenspannung herabsetzen. Erfindungsgemäß werden als grenzflächenaktive Stoffe Tenside eingesetzt, insbesondere Tenside mit einem HLB-Wert (HLB= Hydrophilic Lipophilic Balance) über 10. Eine Auflistung geeigneter Stoffe findet sich in Fiedler, Lexikon der Hilfsstoffe, Editio Cantor Verlag Aulendorf, 5. Auflage, Seite 117-121. Geeignete Tenside sind beispielsweise Salze von Fettsäuren wie beispielsweise Natriumdodecylat, Natriumstearat, Natriumoleat oder Natriumpalmitat sowie Salze von Alkylsulfaten wie beispielsweise Natriumlaurylsulfat. Weiterhin eignen sich polyoxyethylierte Sorbitanfettsäureester wie Polysorbat 20 oder ethoxylierte 12-Hydroxystearinsäure oder Polyglycerinfettsäureester. Geeignet sind auch ethoxylierte Derivate von Rizinusöl oder hydriertem Rizinusöl, beispielsweise Umsetzungprodukte von 35 mol Ethylenoxid mit Rizinusöl oder von 40 mol Ethylenoxid mit hydriertem Rizinusöl. Solche Tenside können in Mengen von 0 bis 20 Gew.-%, bevorzugt bis 10 Gew.-% eingesetzt werden.

Zur Herstellung der erfindungsgemäßen Produkte sind Sprühtrocknungsverfahren geeignet, bei denen eine Lösung der Vinyllactampolymere mit Hilfe von Düsen fein zerstäubt wird und anschließend in einem warmen Luftstrom getrocknet wird. Verarbeitet werden vorzugsweise wässrige Lösungen.

Für die Zerstäubung können Einstoffdüsen oder Mehrstoffdüsen verwendet werden. Als Mehrstoffdüsen kommen insbesondere Zweistoffdüsen in Betracht. Entscheidend ist, das kleine Tröpfchen erzielt werden und die getrockneten Teilchen nicht zusammenkleben.

Die Zerstäubung erfolgt bei für den jeweiligen Düsentyp hohem Druck. Bei der Zerstäubung über Einstoffdüsen haben sich Düsendurchmesser von 0,1 bis 3 mm, bevorzugt 0,2 bis 1 mm, besonders bevorzugt 0,4 bis 0,8 mm und Drücke über 8 MPa besonders bewährt, bevorzugt größer 12 MPa , ganz besonders bevorzugt größer 16 MPa . Der Zerstäubungsdruck kann im Falle der Einstoffdüse bis zu 25 MPa betragen. Bei der Zerstäubung über Zweistoffdüsen haben sich Düsendurchmesser (flüssigkeitsseitig) von 0,6 bis 10 mm, bevorzugt 0,8 bis 3 mm, besonders bevorzugt 1 bis 3 mm und Drücke des Zerstäubungsgases über 0,2 MPa , besonders bevorzugt über 0,4 MPa, ganz besonders bevorzugt über 0,6 MPa bewährt. Der Druck des Zerstäubungsgases kann bis zu 1 MPa betragen. Als Zerstäubungsgase eignen sich die gleichen Gase wie sie zur Trocknung eingesetzt werden.

Die Feststoffkonzentrationen der zu versprühenden Lösungen liegen zwischen 1 und 35 Gew.-%, bevorzugt zwischen 3 und 25 Gew.-% und besonders bevorzugt zwischen 5 und 15 Gew.-%.

Gemäß einer bevorzugten Ausführungsform erfolgt eine Vorerwärmung der Sprühlösung auf Temperaturen von 40 - 180°C.

Die Zerstäubung kann in jedem Sprühturm herkömmlicher Bauart erfolgen. Als Trocknungsgase können Luft oder inerte Gase wie Stickstoff, Argon oder Helium verwendet werden, welche im Gleichstrom oder im Gegenstrom zu den Flüssigkeitströpfchen durch den Trocknungsturm geleitet werden können. Vorzugsweise wird das Trocknungsgas im Gleichstrom eingesetzt. Die Turmeingangstemperatur des Trocknungsgases liegt bei 80 bis 250, vorzugsweise bei 100 bis 200°C. Die Turmausgangstemperatur liegt bei 40 bis 130, vorzugsweise bei 50 bis 110°C.

Die Verdampfung des Lösungsmittels kann sowohl bei Atmosphärendruck als auch bei leichtem Über- oder Unterdruck (+/- 0,01 MPa) erfolgen. Das entstehende Pulver kann beispielsweise über ein Zyklon oder ein Filter von dem Gasstrom abgetrennt werden.

Überraschenderweise wurde gefunden, dass Bindemittel auf Basis von Vinyllactampolymere mit besonderer Form der Teilchen über stark verbesserte Bindemittelaktivitäten verfügen.

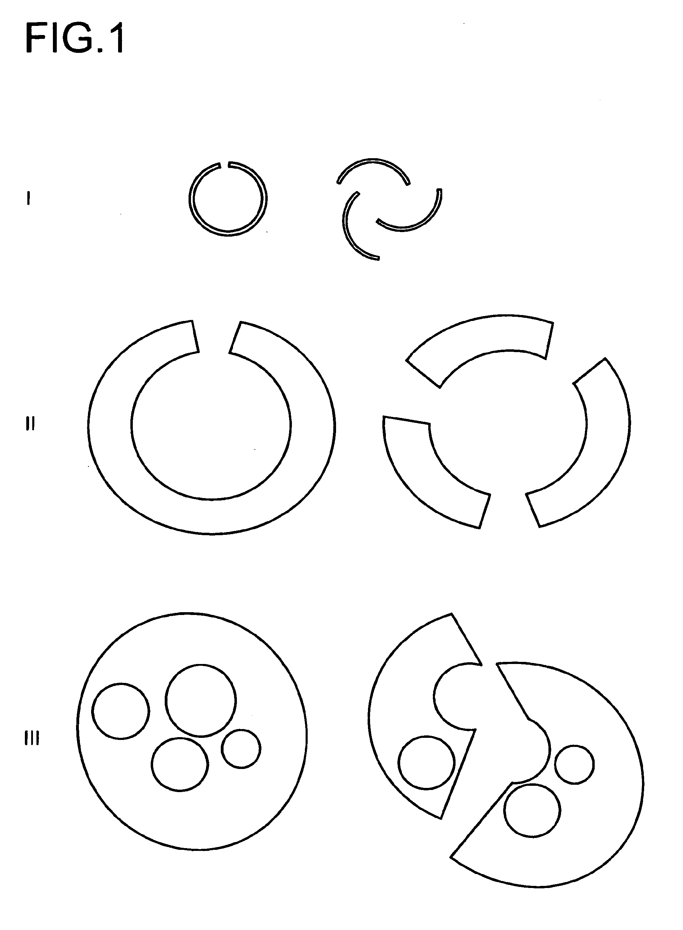

In der Figur sind erfindungsgemäße Teilchen (I) und Teilchen gemäß Stand der Technik (kommerziell erhältliche Produkte; II: Kollidon VA 64; III: Plasdone S 630) schematisch dargestellt.

Um die Teilchengröße der erfindungsgemäßen Bindemittel gewünschtenfalls vor der Verarbeitung weiter zu verkleinern, können auf diese Weise gesprühte Produkte mit den üblichen Mühlen wie z. B. Luftstrahlmühlen, Stiftmühlen gemahlen werden. Durch Mahlen kann die mittlere Teilchengröße beispielsweise auf Werte von 1 bis 20 µm eingestellt werden.

Die Anwendung der erfindungsgemäßen Produkte erfolgt in der Regel durch Mischen mit den übrigen Rezepturbestandteilen und anschließendes Komprimieren zu einer Tablette oder einem Kompaktat. Dabei ist entscheidend, dass das Trockenbindemittel gleichmäßig in der Mischung verteilt ist. Gemäß einer besonderen Ausführung kann auch nach dem Mischen noch Wasser, Dampf oder ein organisches Lösungsmittel zugesetzt werden, wodurch sich die kleinen Teilchen anlösen und zu einer starken Festigkeit der Tablette oder des Kompaktats führen.

Die Herstellung einer Tablette erfolgt üblicherweise auf einer Tablettenpresse, die Herstellung eines Kompaktats auf einem Walzenkompaktor. Zur weiteren Verarbeitung wird das Kompaktat wieder zerkleinert zu einem Granulat, das mit weiteren Zuschlagsstoffen versetzt werden kann und z. B. zu einer Tablette verpresst werden kann. Das Verfahren der Walzenkompaktierung wird auch als Trockengranulierung bezeichnet.

Die Verpressung zu Tabletten kann bei Pressdrücken von bis 800 MPa erfolgen.

Die mit Hilfe der erfindungsgemäßen Bindemittel erhaltenen Tabletten weisen eine hohe Festigkeit auf. Die Festigkeit kann von 40 bis 600 N betragen.

Der Anteil des Trockenbindemittels in der Rezeptur sollte bei 0,5 - 20 Gew.-%, bevorzugt bei 1 - 15 Gew.-% und besonders bevorzugt bei 2 - 12 Gew.-% liegen. Dadurch, dass die erfindungsgemäßen Trockenbindemittel über enorme Bindeeigenschaften verfügen, ist auch die Komprimierung schlecht pressbarer Wirkstoffe und Hilfsstoffe möglich, vor allem wenn diese auch noch in hoher Konzentration vorliegen.

Die erfindungsgemäßen Bindemittel besitzen selbst aufgrund der feinen Teilchen eine schlechtere Fließfähigkeit. So sollte auch in der Mischung mit anderen Bestandteilen eine schlechtere Fließfähigkeit resultieren. Überraschenderweise ergeben sich jedoch in diesen Fällen bessere Fließfähigkeiten.

Häufig sind Bindemittel klebrige Stoffe, die die Ausstosskräfte beim Tablettieren erhöhen, wodurch zahlreiche Probleme verursacht werden können, wie z. B. reduzierte Festigkeit der Tablette, Deckeln, starke Erwärmung der Presswerkzeuge und der Matrizenwand, erhöhter Verschleiß der Presse etc. Völlig unerwartet zeigen die erfindungsgemäßen Bindemittel eine Schmiermittelwirkung, denn die Rest-und Ausstoßkräfte beim Tablettieren sind deutlich niedriger als ohne Verwendung eines Bindemittels oder mit Verwendung eines herkömmlichen Bindemittels.

Für die besondere Bindemittelwirkung, die sich in einer hohen Bruchfestigkeit und niedrigen Friabilität der Tabletten zeigt, spielt der Herstellungsprozess und damit auch die bevorzugte hohlkugelartige oder schalenartige Struktur eine entscheidende Rolle. So weisen Produkte die durch Mahlung erzeugt wurden bei gleicher mittlerer Teilchengröße eine erheblich niedrigere Bindemittelwirkung auf.

Überraschenderweise kann trotz einer höheren mechanischen Festigkeit der Tabletten keine Verlängerung der Zerfallszeit festgestellt werden. Insgesamt ist die Zerfallszeit von Tabletten mit den erfindungsgemäßen Bindemitteln schnell.

Zusammengefasst führen die erfindungsgemäßen Bindemittel zu Tabletten mit außergewöhnlichen mechanischen Eigenschaften, sie ermöglichen die Verpressung nicht oder schwerpressbarer Arzneistoffe, sie ermöglichen eine Reduktion der Gesamttablettenmasse bzw. des Tablettenvolumens und sie sorgen für einen reibungslosen Verlauf des Tablettierprozesses.

Die erfindungsgemäßen Bindemittel eignen sich insbesondere zur Herstellung von Tabletten der folgenden üblicherweise schwer verpressbaren pharmazeutischen Wirkstoffe:
Paracetamol, Carbamazepin, Acetylsalicylsäure, Ascorbinsäure, Metoprololtartrat, Ibuprofen, Pseudoephedrin-HCI, Diphenhydramin-HCl, Dimenhydrinat, Indometacin, Diclofenac-Natrium, N-Acetylcystein, Albendazol, Alpha-Methyldopa, Aluminiumhydroxide, Magnesiumsilikat, Ampicillin, Atenolol-HCI, Captopril, Cimetidin, Diltiazem, Griseofulvin, Levamisol, Magaldrat, Magnesiumcarbonat, Mebendazol, Meprobamat, Metamizol, Metronidazol, Neomycinsulfat, Oxytetracyclin-HCI, Nitrofurantoin, Nystatin, Nicotinsäure, Phenytoin, Piroxicam, Pyrazinamid, Ranitidin, Tetracyclin, Amoxicillin, Chloroquindiphosphat, Ethambutol, Gemfibrozil, Mefenaminsäure, Metformin-HCI, Nalidixinsäure, Naproxen, Probenecid, Rifampicin, Sulfadiazin, Sulfadimidin, Sulfadoxin, Sulfamethoxazol, Sulfathiazol, Valproinsäure, Verapamil, Aciclovir, Allopurinol, Bezafibrat, Carbidopa, Cefuroxim, Cephachlor, Ciprofloxacin, Fenofibrat, Alpha-Liponsäure, Pentoxyfyllin, Piracetam, Propafenon-HCI, Roxithromycin, Sotalol, Sulpirid, Tramadol, Tilidin.

### Beispiele

Als Sprühtrockner wurde ein Technikums-Sprühturm der Firma Niro, Typ Minor (Beispiele 1,3-8, 12, 13) oder ein Produktions-Sprühturm der Firma Zimmerling, Durchmesser 7 m (Beispiel 2) verwendet.

Soweit nicht anders angegeben beziehen sich Prozentangaben auf Gewichtsprozente.

### Beispiel 1

Eine 10 %ige wässrige Lösung von Kollidon K30, einem Polyvinylpyrrolidon mit einem K-Wert von 30 (gemessen in einer 1 gew.-%igen Lösung), wurde in einem Sprühtrockner bei einer Zulufttemperatur von 170°C getrocknet. Die Sprühlösung wurde vor der Zerstäubung auf 80°C erwärmt und die Zerstäubung in feine Tröpfchen erfolgte mittels einer Einstoffdüse (Durchmesser0.5 mm) bei 16 MPa Druck. Die Ablufttemperatur betrug 85°C.

Es wurde ein feines Pulver mit einer mittleren Korngröße von 17µm und einer Schüttdichte von 0,12 g/ml erhalten.

Die mikroskopische Untersuchung zeigte das Vorliegen von hohlkugelförmigen Körpern und Bruckstücken von solchen Körpern (Schalen) auf.

### Beispiel 2

Eine 10 %ige wässrige Lösung von Kollidon VA 64, einem Copolymer aus N-Vinylpyrrolidon(VP) und Vinylacetat (VAc) im Gewichtsverhältnis 6:4, K-Wert 28 (gemessen 1 Gew.-%ig in Wasser) wurde in einem Sprühtrockner bei einer Zulufttemperatur von 150°C getrocknet. Die Sprühlösung wurde vor der Zerstäubung auf 80°C erwärmt und die Zerstäubung in feine Tröpfchen erfolgte mittels einer Einstoffdüse (Durchmesser 1.2 mm)bei 18 MPa Druck. Die Ablufttemperatur betrug 72°C.

Es wurde ein feines Pulver mit einer mittleren Korngröße von 15 µm und einer Schüttdichte von 0,10g/ml erhalten.

Die mikroskopische Untersuchung zeigte das Vorliegen von Hohlkugeln und Bruckstücken von Hohlkugeln (Schalen) mit Wanddicken von1.0.µm auf.

### Beispiel 3

Es wurde eine Lösung von 10% Kollidon 30 (Polyvinylpyrrolidon, K-Wert 30)und 0,3% Triethylcitrat in Wasser hergestellt und in einem Sprühtrockner bei einer Zulufttemperatur von 145°C getrocknet. Die Sprühlösung wurde vor der Zerstäubung auf 75°C erwärmt und die Zerstäubung in feine Tröpfchen erfolgte mittels einer Einstoffdüse (Durchmesser0.5 mm) bei 16 MPa Druck. Die Ablufttemperatur betrug 71°C.

Es wurde ein feines Pulver mit einer mittleren Korngröße von 16µm und einer Schüttdichte von 0,11 g/ml erhalten.

Die mikroskopische Untersuchung zeigte das Vorliegen von Hohlkugeln und Bruckstücken von Hohlkugeln (Schalen) mit Wanddicken von 1.2 µm auf.

### Beispiel 4

Es wurde eine Lösung von 10 % Kollidon 30 und 0,5 % Polysorbat 80 in Wasser hergestellt und in einem Sprühtrockner bei einer Zulufttemperatur von 145°C getrocknet. Die Sprühlösung wurde vor der Zerstäubung auf 75°C erwärmt und die Zerstäubung in feine Tröpfchen erfolgte mittels einer Einstoffdüse (Durchmesser 0.4 mm) bei 16 MPa Druck. Die Ablufttemperatur betrug 71 °C.

Es wurde ein feines Pulver mit einer mittleren Korngröße von 13µm und einer Schüttdichte von 0,11 g/ml erhalten.

Die mikroskopische Untersuchung zeigte das Vorliegen von Hohlkugeln und Bruckstücken von Hohlkugeln (Schalen) mit Wanddicken von 0.9 µm auf.

### Beispiel 5

Es wurde eine Lösung von 5 % Kollidon VA 64 und 0,1% Natriumlaurylsulfat in Wasser hergestellt und in einem Sprühtrockner bei einer Zulufttemperatur von 165°C getrocknet. Die Sprühlösung wurde vor der Zerstäubung auf 78°C erwärmt und die Zerstäubung in feine Tröpfchen erfolgte mittels einer Einstoffdüse (Durchmesser 0.4 mm) bei 20 MPa Druck. Die Ablufttemperatur betrug 79°C.

Es wurde ein feines Pulver mit einer mittleren Korngröße von 13µm und einer Schüttdichte von 0,09 g/ml erhalten.

Die mikroskopische Untersuchung zeigte das Vorliegen von Hohlkugeln und Bruckstücken von Hohlkugeln (Schalen) mit Wanddicken von 0.8 µm auf.

### Beispiel 6

Eine 10 %ige wässrige Lösung eines Copolymers aus N-Vinylpyrrolidon und N-Vinylcaprolactam im Gewichtsverhältnis1:1 (K-Wert 65, gemessen 1 Gew.-%ig in Wasser) wurde in einem Sprühtrockner bei einer Zulufttemperatur von 170°C getrocknet. Die Sprühlösung wurde vor der Zerstäubung auf 80°C erwärmt und die Zerstäubung in feine Tröpfchen erfolgte mittels einer Einstoffdüse (Durchmesser 0.4. mm) bei 18,5 MPa Druck. Die Ablufttemperatur betrug 83°C.

Es wurde ein feines Pulver mit einer mittleren Korngröße von 19 µm und einer Schüttdichte von 0,12 g/ml erhalten.

Die mikroskopische Untersuchung zeigte das Vorliegen von Hohlkugeln und Bruckstücken von Hohlkugeln (Schalen) mit einer Wanddicke von 1.4 µm auf.

### Beispiel 7

Eine 12 gew.-%ige wässrige Lösung eines Copolymers aus Vinylpyrrolidon und Vinyllaurat (mittleres Molekulargewicht 20.000 Dalton, K-Wert 19) im Gewichtsverhältnis 90:10 wurde in einem Sprühtrockner bei einer Zulufttemperatur von 160°C getrocknet. Die Sprühlösung wurde vor der Zerstäubung auf 90°C erwärmt und die Zerstäubung in feine Tröpfchen erfolgte mittels einer Einstoffdüse (Durchmesser 0.5 mm) bei 16 MPa Druck. Die Ablufttemperatur betrug 77°C.

Es wurde ein feines Pulver mit einer mittleren Korngröße von 16µm und einer Schüttdichte von 0,11 g/ml erhalten.

Die mikroskopische Untersuchung zeigte das Vorliegen von Hohlkugeln und Bruchstücken von Hohlkugeln (Schalen) mit einer Wanddicke von1.0 µm auf.

### Beispiel 8

Es wurde eine Lösung von 10 % Kollidon VA 64 und 0,25 % Cremophor RH 40 (Umsetzungsprodukt von hydriertem Rizinusöl mit 45 mol Ethylenoxid) in Wasser hergestellt und in einem Sprühtrockner bei einer Zulufttemperatur von 165°C getrocknet. Die Sprühlösung wurde vor der Zerstäubung auf 78°C erwärmt und die Zerstäubung in feine Tröpfchen erfolgte mittels einer Einstoffdüse (Durchmesser 0.4 mm) bei 20 MPa Druck. Die Ablufttemperatur betrug 79°C.

Es wurde ein feines Pulver mit einer mittleren Korngröße von 13µm und einer Schüttdichte von 0,10 g/ml erhalten.

Die mikroskopische Untersuchung zeigte das Vorliegen von Hohlkugeln und Bruchstücken von Hohlkugeln (Schalen) mit einer Wanddicke von 1.0 µm auf.

### Beispiel 9

Prüfung der Bindemittelwirkung in einer Ascorbinsäurerezeptur

2,00 kg Ascorbinsäure, 2,31 kg Ludipress® (coprozessiertes Produkt aus 93,0 % Lactose, 3,5 % Povidon und 3,5 % Crospovidon), 0,50 kg Bindemittel, 0,15 kg Crospovidon (Kollidon CL, BASF), 0,0125 kg hochdisperse Kieselsäure (Aerosil 200, Degussa) und 0,025 kg Magnesiumstearat wurden durch ein 0,8 mm Sieb in einem Turbulamischer gegeben und 10 min gemischt. Diese Mischung wurde auf einer instrumentierten Excenterpresse (EKO, Fa. Korsch) zu biplanenTabletten mit 12 mm Durchmesser und 500 mg Gesamtgewicht gepresst. Die Presskraft betrug 18kN.

Folgende Bindemittel wurden geprüft:
Produkt aus Beispiel 2
Produkt aus Beispiel 5
Produkt aus Beispiel 8
Kollidon VA 64 Handelware BASF, mittlere Teilchengröße 54 µm, Schüttdichte 0.26 g/ml
Plasdone S 630 (Copolymer aus VP/VAc 6.4) Handelsware ISP, mittlere Teilchengröße 64µm, Schüttdichte 0.23 g/ml
Gemahlenes Kollidon VA 64 Handelware BASF, mittlere Teilchengröße 18 µm

| Bindemittel | Böschungswinkel (°) | Bruchfestigkeit (N) | Ausstoßkraft (N) |
|---|---|---|---|
| Ohne Bindemittel | 38,8 | 50 | 610 |
| Beispiel 2 | 34,4 | 146 | 290 |
| Beispiel 5 | 34,0 | 182 | 310 |
| Beispiel 8 | 34,8 | 165 | 260 |
| Kollidon 64 Handelsware | 37,1 | 96 | 850 |
| Plasdone S 630 Handelsware | 37,3 | 94 | 910 |
| Gemahlenes Kollidon VA 64 | 36,1 | 112 | 820 |

### Beispiel 10

Prüfung der Bindemittelwirkung in einer Paracetamolrezeptur

2,5 kg Paracetamol, 0,655 kg mikrokristalline Cellulose, 0,225 kg Bindemittel, 0,105 kg Crospovidon (Kollidon CL, BASF), 0,025 kg hochdisperse Kieselsäure (Aerosil 200) und 0,015 kg Magnesiumstearat wurden durch ein 0,8 mm Sieb gesiebt und in einem Turbula-Mischer 20 min gemischt. Anschließend wurde die Mischung auf einer Rundläufertablettenpresse ( Korsch PH 106) zu Tabletten mit einem Durchmesser von 16 mm und 705 mg Gesamtgewicht verpresst. Die Presskraft betrug 10kN.

Folgende Bindemittel wurden geprüft:

### Produkt aus Beispiel 2

**Kollidon VA 64 Handelware BASF, mittlere Teilchengröße 54µm**

| Bindemittel | Bruchfestigkeit (N) | Friabilität (%) | Zerfall (Sek.) |
|---|---|---|---|
| Beispiel 2 | 94 | 0,4 | 9 |
| Kollidon 64 Handelsware | 56 | 2,0 | 12 |

### Beispiel 11

Prüfung der Bindemittelwirkung in einer Ibuprofenrezeptur

4,0 kg Ibuprofen, 0,3 kg mikrokristalline Cellulose, 0,3 kg Bindemittel, 0,2 kg Crospovidon (Kollidon CL, BASF), 0,06kg hochdisperse Kieselsäure (Aerosil 200) und 0,03kg Magnesiumstearat wurden durch ein 0,8 mm Sieb gesiebt und in einem Diosna-Mischer 10min gemischt. Anschließend wurde die Mischung auf einer Rundläufertablettenpresse ( Korsch PH 106) zu Tabletten mit einem Durchmesser von 12 mm und 489 mg Gesamtgewicht verpresst. Die Presskraft betrug 9 kN.

### Folgende Bindemittel wurden geprüft:

### Produkt aus Beispiel 5

**Kollidon VA 64 Handelware BASF, mittlere Teilchengröße 57µm**

| Bindemittel | Bruchfestigkeit (N) | Friabilität (%) | Zerfall (min.) |
|---|---|---|---|
| Beispiel 5 | 92 | <0,1 | 9 |
| Kollidon 64 Handelsware | 63 | 0,4 | 13 |

### Beispiel 12

Eine 18 %ige wässrige Lösung von Polyvinylcaprolactam (K-Wert 23.5, 5 %ig in Ethanol) wurde in einem Sprühtrockner bei einer Zulufttemperatur von 135°C getrocknet. Die Zerstäubung in feine Tröpfchen erfolgte mittels Zweistoffdüsen (Durchmesser: Flüssigkeitszufuhr 3 mm, gasseitiger Ringspalt 1 mm) bei 0,4 MPa Gasdruck. Die Ablufttemperatur betrug 74°C.

Es wurde ein feines Pulver mit einer mittleren Korngröße von 15 µm und einer Schüttdichte von 0,10g/ml erhalten.

Die mikroskopische Untersuchung zeigte das Vorliegen von Hohlkugeln und Bruckstücken von Hohlkugeln (Schalen) mit Wanddicken von 1.1 µm auf.

### Beispiel 13

Eine 30%ige wässrige Lösung von Kollidon VA 64 wurde in einem Sprühtrockner bei einer Zulufttemperatur von 153°C getrocknet. Die Sprühlösung wurde vor der Zerstäubung auf 87°C erwärmt und die Zerstäubung in feine Tröpfchen erfolgte mittels Zweistoffdüsen (Durchmesser: Flüssigkeitzufuhr 2 mm, gasseitiger Ringspalt 1 mm ) bei 0,6 MPa Gasdruck. Die Ablufttemperatur betrug 83°C.

Es wurde ein feines Pulver mit einer mittleren Korngröße von 15 µm und einer Schüttdichte von 0,12 g/ml erhalten.

Die mikroskopische Untersuchung zeigte das Vorliegen von Hohlkugeln und Bruckstücken von Hohlkugeln (Schalen) mit Wanddicken von 1.5 µm auf.

## Patentansprüche

1. Feinteilige, pulverförmige Bindemittel aus Vinyllactampolymeren, wobei die Bindemittel eine mittlere Teilchengröße bis zu 35 µm und eine Schüttdichte kleiner 0.2 g/ml aufweisen.

2. Bindemittel nach Anspruch 1, wobei die Bindemittel in Form von hohlkugelförmigen Körpern oder Teilen solcher hohlkugelförmigen Körper mit einer Wanddicke kleiner 3.0 µm vorliegen und das Verhältnis des Hohlkugeldurchmessers zur Wanddicke größer als 10: 1 beträgt.

3. Bindemittel nach Anspruch 1 oder 2 , wobei die Bindemittel eine mittlere Teilchengröße kleiner 30 µm aufweisen.

4. Bindemittel nach Anspruch 1 oder 2, wobei die Bindemittel eine mittlere Teilchengröße kleiner 20 µm aufweisen.

5. Bindemittel nach einem der Ansprüche 1 bis 4, wobei die Bindemittel eine Wanddicke von kleiner 2.5 µm aufweisen.

6. Bindemittel nach einem der Ansprüche 1 bis 5, wobei die Bindemittel eine Wanddicke von kleiner 2.0 µm aufweisen.

7. Bindemittel nach einem der Ansprüche 1 bis 6, wobei das Verhältnis des Hohlkugeldurchmessers zur Wanddicke größer 12:1 beträgt.

8. Bindemittel nach einem der Ansprüche 1 bis 6, wobei das Verhältnis des Hohlkugeldurchmessers zur Wanddicke größer 15:1 beträgt.

9. Bindemittel nach einem der Ansprüche 1 bis 8, **dadurch gekennzeichnet, dass** die Vinyllactampolymere aus 40 - 100 Gew.-% Vinylpyrrolidon oder Vinylcaprolactam oder Gemischen davon und 0 - 60 % eines Vinylesters einer C₁ - C₂₀-Carbonsäure erhalten werden.

10. Bindemittel nach einem der Ansprüche 1 bis 9, wobei das Vinyllactampolymer aus Vinylpyrrolidon und Vinylacetat erhalten wird.

11. Bindemittel nach einem der Ansprüche1 bis 10, wobei das Vinyllactampolymer aus Vinylpyrrolidon und Vinylacetat in einem Gewichtsverhältnis zwischen 40 : 60 und 80 : 20 erhalten wird.

12. Bindemittel nach einem der Ansprüche1 bis 11, wobei das Vinyllactampolymer aus Vinylpyrrolidon und Vinylacetat in einem Gewichtsverhältnis zwischen 50 : 50 und 70 : 30 erhalten wird.

13. Bindemittel nach einem der Ansprüche1 bis 12, wobei das Vinyllactampolymer aus Vinylpyrrolidon und Vinylacetat in einem Gewichtsverhältnis von 60 : 40 erhalten wird.

14. Bindemittel nach einem der Ansprüche 1 bis 13, wobei das Bindemittel zusätzlich zu dem Vinyllactampolymer Weichmacher oder Tenside oder Gemische davon enthält.

15. Bindemittel nach einem der Ansprüche 1 bis 14, wobei das Bindemittel bis zu 10 Gew.-% eines Weichmachers enthält.

16. Bindemittel nach einem der Ansprüche 1 bis 15, wobei das Bindemittel bis zu 20 Gew.-% eines Tensids enthält.

17. Bindemittel nach einem der Ansprüche 1 bis 16 erhalten durch Versprühen einer Lösung mit einem Feststoffgehalt von 5 bis 35 Gew.-% unter Druck mit Hilfe von Düsen.

18. Verwendung von feinteiligen, pulverförmigen Bindemitteln gemäß einem der Ansprüche 1 bis 17 zur Herstellung von Tabletten mit hoher Festigkeit.

19. Verwendung nach Anspruch 18 , **dadurch gekennzeichnet, dass** die Bindemittel trocken mit anderen Tablettenbestandteilen gemischt und komprimiert werden.

20. Verwendung nach Anspruch 18 oder 19, **dadurch gekennzeichnet, dass** der Anteil des Bindemittels am Gesamtgewicht der Tabletten 0,5 - 20 Gew.-% beträgt.

21. Verwendung nach einem der Ansprüche 18 bis 20, **dadurch gekennzeichnet, dass** der Anteil des Bindemittels am Gesamtgewicht der Tabletten 1 - 15 Gew.-% beträgt.

22. Verwendung nach einem der Ansprüche Anspruch 18 bis 21, **dadurch gekennzeichnet, dass** der Anteil des Bindemittels am Gesamtgewicht der Tabletten 2 bis 12 Gew.-% beträgt.

23. Verwendung nach einem der Ansprüche 18 bis 22, **dadurch gekennzeichnet, dass** das Bindemittel mit den übrigen Tablettenbestandteilen trocken gemischt wird und anschließend tablettiert wird.

24. Verwendung nach einem der Ansprüche 18 bis 23, **dadurch gekennzeichnet, dass** das Bindemittel mit den übrigen Tablettenbestandteilen trocken gemischt, walzenkompaktiert, zerkleinert und anschließend tablettiert wird.

25. Verwendung nach einem der Ansprüche 18 bis 24, **dadurch gekennzeichnet, dass** das Bindemittel mit den übrigen Tablettenbestandteilen gemischt, unter Zugabe von Wasser, Dampf oder eines geeigneten organischen Lösungsmittels weiter gemischt, gegebenenfalls getrocknet, und anschließend tablettiert wird.

26. Verwendung nach einem der Ansprüche 18 bis 25, **dadurch gekennzeichnet, dass** die Bindemittel vor der Verarbeitung zu Tabletten durch Mahlprozesse weiter zerkleinert werden.

27. Verfahren zur Herstellung von feinteiligen, pulverförmigen Bindemitteln aus Vinyllactampolymeren gemäß einem der Ansprüche 1 bis 17 durch Versprühen, **dadurch gekennzeichnet, dass** eine wässrige Lösung des Vinyllactampolymers mit Feststoffgehalten von 1 bis 35 Gew.-% unter Druck mittels Düsen zerstäubt wird und mit einem warmen Luftstrom getrocknet wird, wobei die Zerstäubung mittels Einstoffdüsen bei Drücken größer 8 MPa oder mittels Zweistoffdüsen bei Drücken größer 0,2 MPa erfolgt.

28. Verfahren nach Anspruch 27, **dadurch gekennzeichnet, dass** die Zerstäubung mittels Einstoffdüsen bei Drücken größer 12 MPa erfolgt.

29. Verfahren nach Anspruch 27, **dadurch gekennzeichnet, dass** die Zerstäubung mittels Einstoffdüsen bei Drücken größer 16 MPa erfolgt.

30. Verfahren nach Anspruch 27, **dadurch gekennzeichnet, dass** die Zerstäubung mittels Zweistoffdüsen bei Drücken größer 0,4 MPa erfolgt.

31. Verfahren nach Anspruch 27, **dadurch gekennzeichnet, dass** die Zerstäubung mittels Zweistoffdüsen bei Drücken größer 0,6 MPa erfolgt.

32. Verfahren nach einem der Ansprüche 27 bis 31, **dadurch gekennzeichnet, dass** der Feststoffgehalt der Sprühlösung 3 bis 25 Gew.-% beträgt.

33. Verfahren nach einem der Ansprüche 27 bis 32, **dadurch gekennzeichnet, dass** der Feststoffgehalt der Sprühlösung 5 bis 15 Gew.-% beträgt.

34. Verfahren nach einem der Ansprüche 27 bis 33, **dadurch gekennzeichnet, dass** die Sprühlösung vor der Zerstäubung auf Temperaturen zwischen 40 und 180°C erwärmt wird.

35. Verfahren nach einem der Ansprüche 27 bis 34, **dadurch gekennzeichnet, dass** der Sprühlösung bis zu 10 Gew.-% Weichmacher, bezogen auf den Feststoffgehalt der Sprühlösung, zugesetzt werden.

36. Verfahren nach einem der Ansprüche 27 bis 35 **dadurch gekennzeichnet, dass** der Sprühlösung bis zu 20 Gew.-% einesTensids, bezogen auf den Feststoffgehalt der Sprühlösung, zugesetzt werden.

## Claims

1. A finely divided binder in powder form composed of vinyllactam polymers, where the binder has an average particle size of up to 35 µm and an apparent density of less than 0.2 g/ml.

2. The binder according to claim 1, where the binder is in the form of hollow spheres or parts of such hollow spheres having a wall thickness of less than 3.0 µm, and the ratio of the diameter of the hollow sphere to the wall thickness is greater than 10:1.

3. The binder according to claim 1 or 2, where the binder has an average particle size of less than 30 µm.

4. The binder according to claim 1 or 2, where the binder has an average particle size of less than 20 µm.

5. The binder according to any of claims 1 to 4, where the binder has a wall thickness of less than 2.5 µm.

6. The binder according to any of claims 1 to 5, where the binder has a wall thickness of less than 2.0 µm.

7. The binder according to any of claims 1 to 6, where the ratio of the diameter of the hollow sphere to the wall thickness is greater than 12:1.

8. The binder according to any of claims 1 to 6, where the ratio of the diameter of the hollow sphere to the wall thickness is greater than 15:1.

9. The binder according to any of claims 1 to 8, wherein the vinyllactam polymers are obtained from 40 - 100% by weight of vinylpyrrolidone or vinylcaprolactam or mixtures thereof and 0 - 60% of a vinyl ester of a C₁ - C₂₀-carboxylic acid.

10. The binder according to any of claims 1 to 9, where the vinyllactam polymer is obtained from vinylpyrrolidone and vinyl acetate.

11. The binder according to any of claims 1 to 10, where the vinyllactam polymer is obtained from vinylpyrrolidone and vinyl acetate in a ratio of between 40 : 60 and 80 : 20 by weight.

12. The binder according to any of claims 1 to 11, where the vinyllactam polymer is obtained from vinylpyrrolidone and vinyl acetate in a ratio of between 50 : 50 and 70 : 30 by weight.

13. The binder according to any of claims 1 to 12, where the vinyllactam polymer is obtained from vinylpyrrolidone and vinyl acetate in a ratio of 60 : 40 by weight.

14. The binder according to any of claims 1 to 13, where the binder comprises plasticizers or surfactants or mixtures thereof in addition to the vinyllactam polymer.

15. The binder according to any of claims 1 to 14, where the binder comprises up to 10% by weight of a plasticizer.

16. The binder according to any of claims 1 to 15, where the binder comprises up to 20% by weight of a surfactant.

17. The binder according to any of claims 1 to 16 obtained by atomizing a solution with a solids content of from 5 to 35% by weight under pressure with the aid of nozzles.

18. The use of finely divided binders in powder form according to any of claims 1 to 17 for producing tablets with high strength.

19. The use according to claim 18, wherein the binders are mixed dry with other tablet ingredients and compressed.

20. The use according to claim 18 or 19, wherein the proportion of the binder in the total weight of the tablets is 0.5 - 20% by weight.

21. The use as claimed in any of claims 18 to 20, wherein the proportion of the binder in the total weight of the tablets is 1 - 15% by weight.

22. The use as claimed in any of claims 18 to 21, wherein the proportion of the binder in the total weight of the tablets is from 2 to 12% by weight.

23. The use as claimed in any of claims 18 to 22, wherein the binder is mixed dry with the other tablet ingredients and subsequently tableted.

24. The use as claimed in any of claims 18 to 23, wherein the binder is mixed dry with the other tablet ingredients, roll-compacted, comminuted and subsequently tableted.

25. The use according to any of claims 18 to 24, wherein the binder is mixed with the other tablet ingredients, further mixed with the addition of water, steam or of a suitable organic solvent, dried if appropriate, and subsequently tableted.

26. The use according to any of claims 18 to 25, wherein the binders are further comminuted by grinding processes before processing to tablets.

27. A process for producing finely divided binders in powder form composed of vinyllactam polymers according to any of claims 1 to 17 by atomizing, wherein an aqueous solution of the vinyllactam polymer with solids contents of from 1 to 35% by weight is atomized under pressure by means of nozzles and is dried with a stream of hot air, the atomization being effected by means of single fluid nozzles with pressures of greater than 8 MPa or by means of dual fluid nozzles with pressures of greater than 0.2 MPa.

28. The process according to claim 27, wherein the atomization is effected by means of single fluid nozzles with pressures of greater than 12 MPa.

29. The process according to claim 27, wherein the atomization is effected by means of single fluid nozzles with pressures of greater than 16 MPa.

30. The process according to claim 27, wherein the atomization is effected by means of dual fluid nozzles with pressures of greater than 0.4 MPa.

31. The process according to claim 27, wherein the atomization is effected by means of dual fluid nozzles with pressures of greater than 0.6 MPa.

32. The process according to any of claims 27 to 31, wherein the solids content of the spray solution is from 3 to 25% by weight.

33. The process according to any of claims 27 to 32, wherein the solids content of the spray solution is from 5 to 15% by weight.

34. The process according to any of claims 27 to 33, wherein the spray solution is heated before the atomization to temperatures between 40 and 180°C.

35. The process according to any of claims 27 to 34, wherein up to 10% by weight of plasticizer, based on the solids content of the spray solution, are added to the spray solution.

36. The process according to any of claims 27 to 35, wherein up to 20% by weight of surfactant, based on the solids content of the spray solution, are added to the spray solution.

## Revendications

1. Liant pulvérulent à fines particules constitué de polymères de vinyllactame, le liant présentant une taille de particules moyenne allant jusqu'à 35 µm et une densité en vrac inférieure à 0,2 g/ml.

2. Liant selon la revendication 1, le liant se présentant sous la forme de corps de forme sphérique creuse ou de particules de ces corps de forme sphérique creuse présentant une épaisseur de paroi inférieure à 3,0 µm et le rapport du diamètre des sphères creuses à l'épaisseur de paroi étant supérieur à 10:1.

3. Liant selon la revendication 1 ou 2, le liant présentant une taille moyenne de particules inférieure à 30 µm.

4. Liant selon la revendication 1 ou 2, le liant présentant une taille moyenne de particules inférieure à 20 µm.

5. Liant selon l'une quelconque des revendications 1 à 4, le liant présentant une épaisseur de paroi inférieure à 2,5 µm.

6. Liant selon l'une quelconque des revendications 1 à 5, le liant présentant une épaisseur de paroi inférieure à 2,0 µm.

7. Liant selon l'une quelconque des revendications 1 à 6, dans lequel le rapport du diamètre des sphères creuses à l'épaisseur de paroi est supérieur à 12:1.

8. Liant selon l'une quelconque des revendications 1 à 6, dans lequel le rapport du diamètre des sphères creuses à l'épaisseur de paroi est supérieur à 15:1.

9. Liant selon l'une quelconque des revendications 1 à 8, **caractérisé en ce que** les polymères de vinyllactame sont obtenus à partir de 40 à 100 % en poids de vinylpyrrolidone ou de vinylcaprolactame ou de mélanges de ceux-ci et de 0 à 60 % d'un ester de vinyle d'un acide carboxylique en C₁ à C₂₀.

10. Liant selon l'une quelconque des revendications 1 à 9, dans lequel le polymère de vinyllactame est obtenu à partir de vinylpyrrolidone ou d'acétate de vinyle.

11. Liant selon l'une quelconque des revendications 1 à 10, dans lequel le polymère de vinyllactame est obtenu à partir de vinylpyrrolidone et d'acétate de vinyle en un rapport en poids entre 40:60 et 80:20.

12. Liant selon l'une quelconque des revendications 1 à 11, dans lequel le polymère de vinyllactame est obtenu à partir de vinylpyrrolidone et d'acétate de vinyle en un rapport en poids entre 50:50 et 70:30.

13. Liant selon l'une quelconque des revendications 1 à 12, dans lequel le polymère de vinyllactame est obtenu à partir de vinylpyrrolidone et d'acétate de vinyle en un rapport en poids de 60:40.

14. Liant selon l'une quelconque des revendications 1 à 13, le liant contenant en plus du polymère de vinyllactame, des plastifiants ou des tensioactif ou des mélanges de ceux-ci.

15. Liant selon l'une quelconque des revendications 1 à 14, le liant contenant jusqu'à 10 % en poids d'un plastifiant.

16. Liant selon l'une quelconque des revendications 1 à 15, le liant contenant jusqu'à 20 % en poids d'un tensioactif.

17. Liant selon l'une quelconque des revendications 1 à 16, obtenu par pulvérisation d'une solution présentant une teneur en solides de 5 à 35 % en poids sous pression, à l'aide de buses.

18. Utilisation de liants pulvérulents à fines particules selon l'une quelconque des revendications 1 à 17, pour la production de comprimés à solidité élevée.

19. Utilisation selon la revendication 18, **caractérisée en ce que** le liant est mélangé à sec avec d'autres composants de comprimés et est compressé.

20. Utilisation selon la revendication 18 ou 19, **caractérisée en ce que** la proportion du liant au poids total des comprimés est de 0,5 à 20 % en poids.

21. Utilisation selon l'une quelconque des revendications 18 à 20, **caractérisée en ce que** la proportion du liant au poids total des comprimés est de 1 à 15 % en poids.

22. Utilisation selon l'une quelconque des revendications 18 à 21, **caractérisée en ce que** la proportion du liant au poids total des comprimés est de 2 à 12 % en poids.

23. Utilisation selon l'une quelconque des revendications 18 à 22, **caractérisée en ce que** le liant est mélangé avec le reste des composants de comprimés à sec et est ensuite mis en comprimés.

24. Utilisation selon l'une quelconque des revendications 18 à 23, **caractérisée en ce que** le liant est mélangé à sec avec le reste des composants de comprimés, est compacté au rouleau, broyé et ensuite mis en comprimés.

25. Utilisation selon l'une quelconque des revendications 18 à 24, **caractérisée en ce que** le liant est mélangé avec le reste des composants de comprimés, mélangé encore en ajoutant de l'eau, de la vapeur ou un solvant organique approprié, éventuellement séché et ensuite mis en comprimés.

26. Utilisation selon l'une quelconque des revendications 18 à 25, **caractérisée en ce que** le liant est encore broyé avant la transformation en comprimés par des procédés de broyage.

27. Procédé de fabrication d'un liant pulvérulent à fines particules constitué de polymères de vinyllactame selon l'une quelconque des revendications 1 à 17 par pulvérisation, **caractérisé en ce qu'**une solution aqueuse du polymère de vinyllactame présentant une teneur en solides de 1 à 35 % en poids est pulvérisée sous pression au moyen de buses et est séchée avec un courant d'air chaud, la pulvérisation s'effectuant au moyen de buses unitaires à des pressions supérieures à 8 MPa ou au moyen de buses binaires à des pressions supérieures à 0,2 MPa.

28. Procédé selon la revendication 27, **caractérisé en ce que** la pulvérisation s'effectue au moyen de buses unitaires à des pressions supérieures à 12 MPa.

29. Procédé selon la revendication 27, **caractérisé en ce que** la pulvérisation s'effectue au moyen de buses unitaires à des pressions supérieures à 16 MPa.

30. Procédé selon la revendication 27, **caractérisé en ce que** la pulvérisation s'effectue au moyen de buses binaires à des pressions supérieures à 0,4 MPa.

31. Procédé selon la revendication 27, **caractérisé en ce que** la pulvérisation s'effectue au moyen de buses binaires à des pressions supérieures à 0,6 MPa.

32. Procédé selon l'une quelconque des revendications 27 à 31, **caractérisé en ce que** la teneur en solides de la solution de pulvérisation est de 3 à 25 % en poids.

33. Procédé selon l'une quelconque des revendications 27 à 32, **caractérisé en ce que** la teneur en solides de la solution de pulvérisation est de 5 à 15 % en poids.

34. Procédé selon l'une quelconque des revendications 27 à 33, **caractérisé en ce que** la solution de pulvérisation est chauffée avant la pulvérisation à des températures entre 40 et 180° C.

35. Procédé selon l'une quelconque des revendications 27 à 34, **caractérisé en ce que** l'on ajoute à la solution de pulvérisation, jusqu'à 10 % en poids de plastifiant par rapport à la teneur en solides de la solution de pulvérisation.

36. Procédé selon l'une quelconque des revendications 27 à 35, **caractérisé en ce que** l'on ajoute à la solution de pulvérisation, jusqu'à 20 % en poids d'un tensioactif par rapport à la teneur en solides de la solution de pulvérisation.
